# EUROPEAN PATENT APPLICATION

(11) **EP 1 624 117 A1**
(43) Date of publication of application: **08.02.2006**
(21) Application number: 04425610.5
(22) Date of filing: 05.08.2004
(51) Int. Cl.: E03D 9/02

(54) **Device for supporting sanitizing products for a toilet bowl, and method for producing the device**

(71) Applicant: Deoflor S.p.A., 27029 Vigevano (Pavia) (IT)
(72) Inventor: Ruffina, Laura, 27036 Mortara (IT)
(74) Representative: Modiano, Micaela Nadia

(57) **Abstract**

A device for supporting sanitizing and/or detergent products or the like for toilet bowls, and a method for producing it. The device is made of a synthetic material that incorporates fragrancing substances adapted to be released gradually by the synthetic material.

## Description

The present invention relates to a device for supporting sanitizing and/or detergent products or the like for toilet bowls and to a method for producing the device.

Devices for dispensing fragrancing and detergent and/or sanitizing substances for toilet bowls are known. These devices are generally constituted by a supporting element, which is usually shaped so that it can be hooked onto the rim of the toilet bowl and supports a solid bar or tablet or one or more bottles containing a fragrancing and detergent and/or sanitizing product in gel or liquid form.

The liquid and gel products, as well as the solid bars or tablets, are constituted by a mixture of surfactants, inert fillers, acidity or viscosity adjusters, bleaching agents, rinse aids, sanitizing substances or scale removers, fragrance, and colors.

In these products, the fragrance can cause incompatibilities with some of the raw materials used to prepare the detergent and/or sanitizing components, particularly if they contain bleaching agents based on chlorine or bleach. For this reason, in formulating such products it is necessary to perform a certain number of compatibility tests and to select the fragrances to be used, limiting the range of available fragrances.

This inevitably entails a limitation of the fragrances that can be used in the production of such products.

Moreover, the fragrancing action of such products, particularly in the case of solid bars or tablets, is not linear but is stronger at the beginning of the period of use and gradually decreases during consumption of the product, until it practically disappears often before the product is used up.

The aim of the present invention is to solve the problems described above by providing a device for supporting sanitizing and/or detergent products or the like for toilet bowls that allows to render the fragrancing action independent of the detergent and/or sanitizing action in devices for fragrancing and cleaning and/or sanitizing toilet bowls.

Within this aim, an object of the invention is to provide a device that eliminates compatibility problems between the fragrancing substances and the detergent substances that can be used for this particular application.

Another object of the invention is to provide a device which makes possible to achieve a fragrancing effect that is substantially constant over time, regardless of the consumption of the detergent and/or sanitizing product.

Another object of the invention is to provide a device that avoids releasing environment-polluting fragrancing substances into the waste water of the toilet bowl.

This aim and these and other objects that will become better apparent hereinafter are achieved by a device for supporting sanitizing and/or detergent products or the like for toilet bowls, characterized in that it is made of a synthetic material that incorporates fragrancing substances that are adapted to be gradually released by said synthetic material.

This aim and these and other objects are also achieved by a method for producing a device as described above, the method comprising the step of incorporating at least one fragrance in at least one synthetic material.

Further characteristics and advantages of the invention will become better apparent from the description of a preferred but not exclusive embodiment of the supporting device according to the invention, described hereinafter by way of non-limiting example.

The supporting device according to the invention, which may have a structure and a configuration that are similar to those of the supporting devices currently used to support solid bars or tablets or one or more dispensing devices that contain a fragrancing and detergent and/or sanitizing product in gel or liquid form, has the particularity that it is made of a synthetic material that incorporates fragrancing substances adapted to be gradually released by the synthetic material.

The supporting device according to the invention preferably has engagement means, such as for example a hook-shaped portion, which can engage the rim of the toilet bowl.

In another aspect, the invention relates to a method for producing a device such as the one described above, which comprises the step of incorporating at least one fragrancing substance in at least one synthetic material of which the device is made.

Adapted synthetic materials are commercially available. Advantageously, the synthetic materials are selected from the group that comprises polyethylene, low-density polyethylene, polypropylene, ethylene-vinyl acetate copolymers, ethyl acrylate polymers, rubber, thermoplastic polyurethanes, polyamides, ABS and mixtures thereof. Low-density polyethylene is preferred because its lower apparent density with respect to polyethylene allows to incorporate, for an equal weight, a larger quantity of fragrancing substances.

Any fragrancing substance is suitable for carrying out the invention. In particular, it is possible to use substances used in the field of ambient fragrancing. Preferably, the fragrancing substances are selected from the group that comprises one or more fragrancing substances that are high-boiling and have a low evaporation curve, where "high-boiling" and "low evaporation curve" indicate substances that evaporate at temperatures above 50 °C.

Advantageously, the fragrancing substance is added to the synthetic material before and/or during the step of working said synthetic material so as to provide the device according to the invention. Preferably, the fragrancing substance is incorporated in the synthetic material, where said synthetic material is in the form of granules.

In a preferred embodiment, the incorporation step comprises cold impregnation of the synthetic material with the fragrancing substance, advantageously by mechanical agitation. In this embodiment, the most preferred synthetic materials are ethylene-vinyl acetate copolymers. Cold impregnation advantageously occurs before the step of molding or extruding the synthetic material, in a time comprised between 1 and 24 hours, preferably for a time of 2 hours, at a temperature below 40 °C, preferably at room temperature. The amount of fragrancing substance that can be incorporated by cold impregnation is at least 30% by weight on the total weight of the synthetic fragrancing substance, preferably between 30 and 40%.

In a particularly preferred embodiment, during the cold impregnation step one or more fragrancing accelerator substances are also added in quantities up to 50% by weight on the total weight of the fragrancing substance. These accelerators are preferably terpene extracts. Cold impregnation is advantageous because it leaves the synthetic material perfectly dry, thus leaving unchanged the chemical and organoleptic characteristics of the fragrance, avoiding its thermal degradation.

In a different embodiment, the incorporation step comprises direct injection of the fragrancing substance during extrusion of the synthetic material.

Regardless of the method used to perform the incorporation step, the synthetic material with the fragrancing substance added thereto is advantageously worked, for example by molding or extrusion, together with other optional agents, such as resins and/or elastomers, which are particularly adapted to improve the performance of the device in terms of flexibility, strength and tactile impact. Preferred optional agents are selected from polypropylene, high-density polyethylene (HDPE), low-density polyethylene (LDPE) and mixtures thereof.

In practice it has been observed that the supporting device according to the invention fully achieves the intended aim and objects, since it allows to render the detergent and/or sanitizing effect independent of the fragrancing effect in devices to be placed in toilet bowls, leaving greater freedom in formulating the fragrancing substances and the detergent and/or sanitizing substances and allowing the use of fragrances that would not be compatible with the detergent and/or sanitizing substances.

Moreover, thanks to the fact that the fragrancing substances that impregnate the supporting device according to the invention are not removed by the flushing water but are diffused directly into the environment, it is possible to use without problems fragrancing substances that might be waste water pollutants.

The device and the method for producing it thus conceived are susceptible of numerous modifications and variations, all of which are within the scope of the inventive concept; all the details may further be replaced with other technically equivalent elements.

## Claims

1. A device for supporting sanitizing and/or detergent products or the like for toilet bowls, **characterized in that** it is made of a synthetic material that incorporates fragrancing substances adapted to be gradually released by said synthetic material.

2. The device according to claim 1, **characterized in that** it has engagement means that can engage the rim of the toilet bowl.

3. A device for dispensing sanitizing and/or detergent substances or the like in toilet bowls, comprising a supporting device and at least one product that contains sanitizing and/or detergent substances, **characterized in that** said supporting device is made of a synthetic material that incorporates fragrancing substances that are adapted to be gradually released by said synthetic material, according to one or more of the preceding claims.

4. The device according to claim 3, **characterized in that** said at least one product containing sanitizing and/or detergent substances is devoid of fragrancing substances.

5. A method for producing a device according to one or more of the preceding claims, **characterized in that** it comprises the step of incorporating at least one fragrancing substance in at least one synthetic material.

6. The method according to claim 5, further comprising a step of molding or extruding the synthetic material, wherein said incorporation step is carried out beforehand or simultaneously with respect to said molding or extrusion step.

7. The method according to claim 5, wherein the molding or extrusion step is carried out in the presence of at least one resin and/or elastomer, selected from the group that consists of polypropylene, high-density polyethylene, and low-density polyethylene.

8. The method according to claim 5, **characterized in that** said synthetic material is in granular form.

9. The method according to claim 5, wherein said incorporation step comprises cold impregnation of said at least one synthetic material with said at least one fragrancing substance.

10. The method according to claim 9, wherein said cold incorporation step occurs by mechanical agitation and before the molding or extrusion step.

11. The method according to claim 9, wherein said cold incorporation step is carried out over a time between 1 and 24 hours and at a temperature below 40 °C.

12. The method according to claim 9, wherein said cold impregnation step also comprises addition of one or more fragrancing accelerators selected among terpene extracts.

13. The method according to claim 9, wherein the quantity of the at least one fragrancing substance is at least 30%, preferably between 30 and 40%, by weight on the total weight of the synthetic fragrancing substance.

14. The method according to claim 9, wherein said incorporation step comprises the direct injection of the at least one fragrancing substance during extrusion of the synthetic material.

15. The invention according to claims 1 and 5, **characterized in that** said synthetic material is selected from the group that consists of polyethylene, low-density polyethylene, polypropylene, ethylene-vinyl acetate copolymers, ethyl acrylate polymers, rubber, thermoplastic polyurethanes, polyamides, ABS and mixtures thereof.

16. The invention according to claims 1 and 5, **characterized in that** said fragrancing substances are selected from the group that consists of one or more fragrancing substances with a high boiling point and a low evaporation curve.
